# EUROPÄISCHE PATENTSCHRIFT

(11) **EP 0 711 545 B1**
(45) Veröffentlichungstag und Bekanntmachung des Hinweises auf die Patenterteilung: **29.12.1999**
(21) Anmeldenummer: 95116841.8
(22) Anmeldetag: 26.10.1995
(51) Int. Cl.: A61K 7/50, C11D 1/90

(54) **Milde, wässrige, tensidische Zubereitungen für kosmetische Zwecke und Reinigungsmittel**
Aqueous mild surfactants containing preparations for cosmetic and cleaning use
Préparations aqueuses de tensioactif doux pour usage cosmétique et nettoyage

(30) Priorität: 07.11.1994 DE 4439642
(43) Veröffentlichungstag der Anmeldung: 15.05.1996
(73) Patentinhaber: Th. Goldschmidt AG, 45127 Essen (DE)
(72) Erfinder: Grüning, Burghard, Dr., D-45134 Essen (DE); Weitemeyer, Christian, Dr., D-45134 Essen (DE)

(56) Entgegenhaltungen:
- EP-A- 0 415 142
- EP-A- 0 417 501
- EP-A- 0 522 157
- EP-A- 0 563 747
- DATABASE WPI Week 9426 Derwent Publications Ltd., London, GB; AN 94-211030 & JP-A-06 145 693 (KAWAKEN FINE CHEM CO LTD) , 27.Mai 1994
- DATABASE WPI Week 8621 Derwent Publications Ltd., London, GB; AN 86-133800 & JP-A-61 069 713 (DUSKIN KK) , 10.April 1986

## Beschreibung

Die Erfindung betrifft milde, wäßrige, tensidische Zubereitungen für kosmetische Zwecke und Reinigungsmittel.

Betaine und als solche vor allem Amidopropylbetaine werden als amphotere Tenside insbesondere für Haar- und Hautreinigungspräparate, wie Shampoos, hautschonende Schaum- und Duschgele, Intim- und Körperpflegemittel eingesetzt. Unter anderem verbessern sie die dermatologischen Eigenschaften anionischer und nichtionischer Tenside und bewirken ein angenehmes Hautgefühl. Darüber hinaus können die Betaine auch mit Vorteil in Reinigungsmitteln, wie Geschirrspülmitteln und Feinwaschmitteln eingesetzt werden.

Als Betaine des Standes der Technik kommen vor allem Fettsäureamidopropylbetaine zum Einsatz, deren Fettsäurereste im Gemisch im allgemeinen 8 bis 18 oder 12 bis 18 Kohlenstoffatome aufweisen. Als besonders wirksam hat sich das Cocosfettsäureamidopropylbetain erwiesen, in dem die Fettsäurereste wie folgt verteilt sein können:

| | |
|---|---|
| Fettsäurerest mit 8 C-Atomen ca. | 7 % |
| Fettsäurerest mit 10 C-Atomen ca. | 6 % |
| Fettsäurerest mit 12 C-Atomen ca. | 49 % |
| Fettsäurerest mit 14 C-Atomen ca. | 19 % |
| Fettsäurerest mit 16 C-Atomen ca. | 9 % |
| Fettsäurerest mit 18 C-Atomen ca. | 10 % |

Wenn auch mit Cocosfettsäureamidopropylbetain milde Tensidformulierungen hergestellt werden können, besitzen doch handelsübliche wäßrige Lösungen des Betains oder deren Verdünnungen mit Wasser ein deutliches Reizpotential, welches sich in Untersuchungen an Haut und Schleimhaut, aber auch in modernen invitro-Testmethoden zur Feststellung der Reizwirkung eines Tensides, wie dem Rote-Blutkörperchen-Test, bestimmen läßt.

Daneben machen ständig zunehmende Anforderungen der Verbraucher an die Milde von kosmetischen Zubereitungen und Reinigungsmitteln die Bereitstellung besonders milder Tenside erforderlich.

Es hat sich deshalb die Aufgabe gestellt, außerordentlich milde und reizarme Zubereitungen zu finden, die dem Anspruch von hoher Hautverträglichkeit genügen.

Überraschenderweise wurden tensidische Zubereitungen gefunden, die als Tenside Betaine der allgemeinen Formel 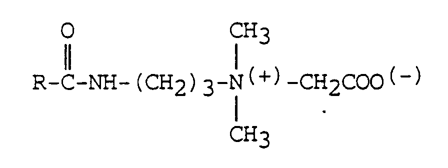 in der R einen Alkylrest mit 7 bis 21 C-Atomen bedeutet, wobei 20 bis 100 Gew.-% der eingesetzten Betaine als Rest R einen Alkylrest mit 7 bis 9 C-Atomen aufweisen,
in einer Menge von 1,0 bis 40 Gew.-%, bezogen auf die Zubereitung, enthalten.

Diese erfindungsgemäßen Zubereitungen sind erheblich milder und reizärmer als die des Standes der Technik und führen nur zu geringen bis zu praktisch keinen Irritationen bei ihrem Einsatz, beispielsweise bei Gesichtswässern, Shampoos oder Lotionen.

Es war überraschend, daß gerade Betaine mit einem relativ kurzkettigen Fettsäurerest diese besonderen milden Eigenschaften zeigen, denn in der Fachliteratur werden gerade solchen Tensiden, die durch kurzkettige Fettsäurereste gekennzeichnet sind, erhöhte reizende Eigenschaften zugeschrieben.

So ist z. B. der Zeitschrift Seifen - Öle - Fette - Wachse - 93. Jg. - Nr. 15/1967 vom 19. Juli 1967 u. a. auf Seite 521 zu entnehmen, daß bei Tensiden mit kürzeren Alkylketten die dermatologischen Eigenschaften weniger zufriedenstellend sind. Deshalb wird auch empfohlen, Tenside mit Alkylketten, die 12 bis 14 bzw. 12 bis 18 Kohlenstoffatome aufweisen, im kosmetischen Sektor einzusetzen.

Im gleichen Sinne lehrt auch die DE-PS 30 11 549, daß Alkylethersulfate, die einen erhöhten Anteil an Alkylresten mit 14 Kohlenstoffatomen aufweisen, mildere Formulierungen ergeben als solche, die im Vergleich dazu einen größeren Anteil an Resten mit 12 und einen geringeren Anteil an Resten mit 14 Kohlenstoffatomen aufweisen.

Es ist zweckmäßig, wenn die erfindungsgemäßen Zubereitungen diese Betaine in einer Menge von 1,5 bis 20 Gew.-%, bezogen auf die Zubereitung, enthalten.

Von Vorteil ist es, wenn 40 bis 100 Gew.-% dieser Betaine einen Alkylrest mit 7 bis 9 C-Atomen aufweisen.

Besonders bevorzugt sind Zubereitungen, wenn mehr als 90 Gew.-% der eingesetzten Betaine einen Alkylrest mit 7 bis 9 C-Atomen aufweisen.

Die erfindungsgemäßen Zubereitungen können anionische Tenside enthalten. Als geeignete anionenaktive Tenside sind insbesondere Alkylethersulfat, weiterhin Monoglyceridsulfate, Alkylethercarbonsäuren und deren Alkalisalze als milde, hautverträgliche Detergentien zu nennen.

Eine weitere bevorzugte Klasse anionischer Tenside sind langkettige Acylsarkosinate bzw. deren Alkalisalze, beispielsweise Natriumlauroylsarkosinat.

Im Rahmen der Erfindung weiterhin geeignete anionische Tenside sind Alphaolefinsulfonate, Alkylsulfate bzw. deren Salze und insbesondere Alkalisalze von Sulfobernsteinsäurehalbestern.

Die erfindungsgemäßen Zubereitungen können weiterhin auch nichtionische Tenside enthalten. Ein bevorzugtes nichtionisches Tensid gehört dabei zu der Klasse der Alkylpolyglucoside der allgemeinen Formel

R-O-(R¹O)ₙ-Zₓ

worin R eine Alkylgruppe mit 8 bis 18 Kohlenstoffatomen, R¹ eine Ethylen- oder Propylengruppe, Z einen Saccharidrest mit 5 bis 6 Kohlenstoffatomen, n eine Zahl von 0 bis 10, und x eine Zahl zwischen 1 und 5 bedeuten.

Obwohl Alkylpolyglucoside der obengenannten allgemeinen Formel als nichtionische Tenside in den erfindungsgemäßen Zusammensetzungen bevorzugt werden, ist es auch möglich, statt dessen oder, vorzugsweise gemeinsam mit denselben, weitere nichtionische Tenside einzusetzen. Als solche sind z. B. Fettsäureester mit mehrwertigen Alkoholen, wie Glycerin, Fettsäurepolyglykolester, Fettalkoholethoxylate, Mischprodukte aus Ethylenoxid und Propylenoxid zu nennen. Weitere nichtionische Tensidbestandteile können sein Aminoxide, Fettsäuremono- oder -dialkanolamide.

Die erfindungsgemäßen milden, wäßrigen, tensidischen Zubereitungen können alle üblichen in solchen Mitteln zum Einsatz gelangenden Stoffe enthalten.

Als solche seien Stabilisatoren, Parfumöle, Verdickungsmittel, die aus der Gruppe der assoziativen Verdickungsmittel oder der Gruppe der polymeren Verdickungsmittel, wie beispielsweise Polyacrylsäure und deren Derivate, Cellulosederivate, Alginate, stammen können, ferner Farbstoffe sowie konditionierende und pflegende Bestandteile, wie z. B. kationische, amphotere Polymere, Lanolinderivate, Cholesterin, Pantothensäure, Polydimethylsiloxane oder deren Derivate, Konservierungsstoffe etc. genannt.

Die Herstellung der erfindungsgemäßen Produkte erfolgt durch Zusammenrühren der einzelnen Komponenten in Wasser, wobei auch Vormischungen verschiedener Bestandteile verwendet werden können.

Anhand der folgenden Vergleichsbeispiele wird die überraschend milde Wirkung der Betaine in erfindungsgemäßen Zubereitungen gezeigt, deren Fettsäurereste sich zu mehr als 20 % aus C₈/C₁₀-Fettsäure ableiten. Die Prozentangaben beziehen sich auf Gewichtsprozent.

### Beispiel 1

Fettsäureamidopropylbetaine, abgeleitet von Fettsäuren unterschiedlicher Kettenlängen, werden mit Hilfe einer in-vitro-Methode, dem Rote-Blutkörperchen-Test (RBC), auf ihre physiologische Verträglichkeit untersucht. Der RBC-Test ist von J. W. Pape und U. Hoppe in der Zeitschrift Arzneim.-Forsch./Drug Res. 40, 498 (1990) beschrieben und wird entsprechend der Beschreibung durchgeführt.

Hierbei werden zur Charakterisierung der typischen irritativen Wechselbeziehung von Tensiden mit intakten Zellstrukturen die Zerstörung der Blutzellen durch Lyse und die Denaturierung des freigesetzten Hämoglobins gemessen. Das Ergebnis stellt der Quotient aus Lyse (L) und Denaturierung (D) L/D dar. Je kleiner der L/D-Wert ist, desto reizender ist die Substanz bzw. Formulierung einzustufen.

Die Ergebnisse sind in Tabelle 1 zusanmengefaßt. Für die zur Anwendung kommenden Fettsäureamidopropylbetaine wird die im folgenden abgekürzte Formulierung benutzt:

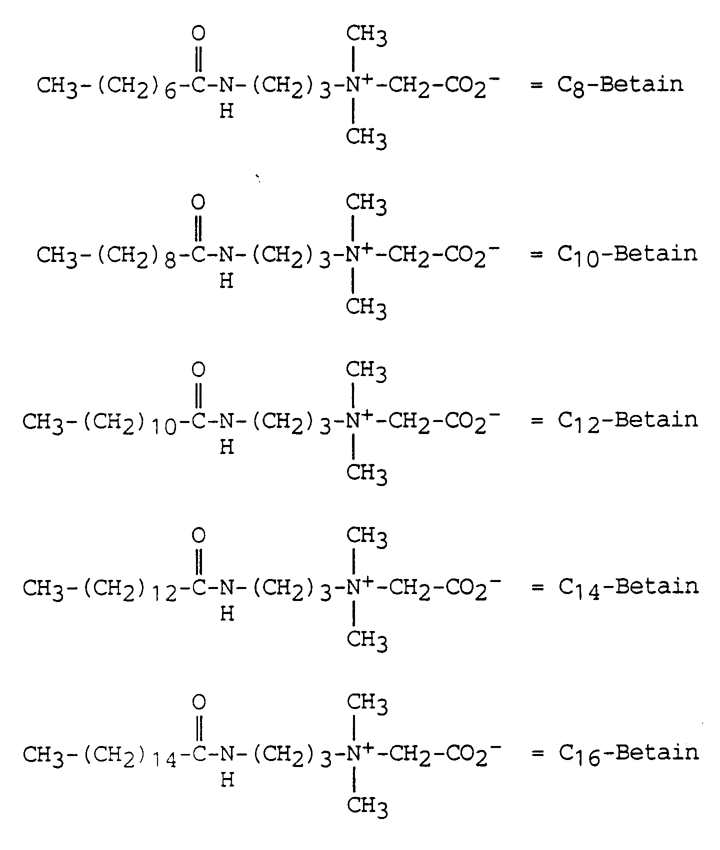

**Tabelle 1**

| Betain | L | D | L/D |
|---|---|---|---|
| C₈-Betain | keine Hämolyse | | >> 100 |
| C₁₀-Betain | keine Hämolyse | | >> 100 |
| C₁₂-Betain | 151 | 17 | 8,9 |
| C₁₄-Betain | 13 | 9,2 | 1,4 |
| C₁₆-Betain | 4,4 | 4,4 | 1,0 |

Die Tabelle zeigt, daß die Reizwirkung der Betaine auf Haut und Schleimhaut mit wachsender Länge der Alkylkette zunimmt, während die kurzkettigen, von der Capryl- und Caprinsäure abgeleiteten Fettsäureamidopropylbetaine praktisch keine Reizwirkung ausüben.

### Beispiel 2

Aus C₈/C₁₀-Betain und Cocosfettsäureamidopropylbetain (Cocobetain) werden milde Tensidformulierungen hergestellt, die sich hinsichtlich ihres Gehaltes an C₈/C₁₀-Betain und Cocobetain unterscheiden. Das C₈/C₁₀-Betain setzt sich zu gleichen Teilen aus C₈-Betain und C₁₀-Betain zusammen. Das Cocobetain ist gekennzeichnet durch folgende Verteilung der Fettsäurereste:

| | |
|---|---|
| C₈-Betain | 7,2 % |
| C₁₀-Betain | 5,8 % |
| C₁₂-Betain | 49,0 % |
| C₁₄-Betain | 18,6 % |
| C₁₆-Betain | 9,1 % |
| C₁₈-Betain | 10,3 % |

In Tabelle 2 sind die Zusammensetzung der Formulierungen und ihre Reizwirkung, bestimmt im RBC-Test, aufgeführt.

**Tabelle 2**

| Formulierung | A | B | C | D | E |
|---|---|---|---|---|---|
| C₈/C₁₀-Betain [%] | 15 | - | 5 | 7,5 | 10 |
| Cocobetain [%] | - | 15 | 10 | 7,5 | 5 |
| C₈/C₁₀-Anteil im Betain [%] | 100 | 13 | 42 | 57 | 72 |
| L | - | 24 | 37 | 51 | 75 |
| D | - | 8,4 | 3,2 | 2,2 | 2,2 |
| L/D | >>100 | 2,9 | 11 | 23 | 34 |

Wie die Ergebnisse zeigen, nimmt mit zunehmendem Gehalt an C₈/C₁₀-Anteilen die Reizwirkung des Betains deutlich ab und seine Verträglichkeit deutlich zu.

### Beispiel 3

Aus C₈/C₁₀-Betain und unten beschriebenen Tensiden werden milde Tensidformulierungen hergestellt. Diese werden verglichen mit analogen Formulierungen, die C₁₂-Betain oder Cocosfettsäureamidopropylbetain zur Grundlage haben. Die Formulierungen enthalten jeweils 10 % Aktivsubstanz Betain und 5 % Aktivsubstanz des zweiten Tensides. Weitere Komponenten außer Wasser sind in den Formulierungen nicht enthalten.

Das C₈/C₁₀-Betain setzt sich, wie in Beispiel 2, zu gleichen Teilen aus C₈-Betain und C₁₀-Betain zusammen. Die Zusammensetzung des Cocosfettsäureamidopropylbetains ist identisch mit der des in Beispiel 2 verwendeten Produktes.

Die neben den Betainen eingesetzten Tenside sind:
Laurylsulfosuccinat-Dinatriumsalz (LSSDNa), im Handel erhältlich unter der Bezeichnung TEGO SULFOSUCCINATE F 30 (Th. Goldschmidt AG)
Natriumlauroylsarcosinat (NaLSC), im Handel erhältlich unter der Bezeichnung Hamposyl L 30 (Grace)
Laurylpolyglucosid (APG), im Handel erhältlich unter der Bezeichnung Plantaren 1200 (Henkel)

In Tabelle 3 sind die Zusammensetzung der Formulierungen und ihre Reizwirkung, bestimmt im RBC-Test, aufgeführt.

**Tabelle 3**

| | LSSDNa | | | NaLSC | | | APG | | |
|---|---|---|---|---|---|---|---|---|---|
| | L | D | L/D | L | D | L/D | L | D | L/D |
| C₈/C₁₀-Betain | *) | 22 | >100 | 563 | 20 | 28 | keine Häm | | >>100 |
| C₁₂-Betain | 49 | 13 | 3,8 | 84 | 18 | 4,7 | 81 | <1 | >100 |
| Cocobetain | 22 | 4,6 | 4,8 | 36 | 12 | 3,0 | 46 | <1 | >100 |
| Häm = Hämolyse, *) = keine vollständige Hämolyse | | | | | | | | | |

Die Ergebnisse machen deutlich, daß Tensidformulierungen, die C₈/C₁₀-Betain zur Grundlage haben, eine deutlich geringere Reizwirkung haben als solche, die auf C₁₂-Betain oder Cocobetain basieren.

### Beispiel 4

Aus C₈/C₁₀-Betain und den drei anderen Tensiden, die in Beispiel 3 beschrieben sind, werden milde Tensidformulierungen hergestellt. Die Formulierungen enthalten 5 bis 10 % C₈/C₁₀-Betain und 10 bis 5 % Laurylsulfosuccinat-Dinatriumsalz (LSSDNa), Natriumlauroylsarcosinat (NaLSC) bzw. Laurylpolyglucosid (APG). Solche, die 10 % C₈/C₁₀-Betain enthalten, sind identisch mit den in Beispiel 3 beschriebenen. Die Formulierungen werden auf ihre physiologische Verträglichkeit mit dem RBC-Test überprüft. Die Ergebnisse sind in Tabelle 4 dargestellt.

**Tabelle 4**

| Formulierung | F | G | H | I | K | L | M | N | O |
|---|---|---|---|---|---|---|---|---|---|
| C₈/C₁₀-Betain [%] | 10 | 7,5 | 5 | 10 | 7,5 | 5 | 10 | 7,5 | 5 |
| LSSDNa [%] | 5 | 7,5 | 10 | - | - | - | - | - | - |
| NaLSC [%] | - | - | - | 5 | 7,5 | 10 | - | - | - |
| APG [%] | - | - | - | - | - | - | - | 7,5 | 10 |
| L | * | * | * | 563 | 409 | 307 | ** | ** | ** |
| D | 22 | 31 | 42 | 20 | 37 | 55 | - | - | - |
| L/D | >100 | >100 | >100 | 28,2 | 11,1 | 5,6 | >>100 | >>100 | >>100 |

| | | | | | | | | | |
|---|---|---|---|---|---|---|---|---|---|
| * = keine vollständige Hämolyse | | | | | | | | | |
| ** = keine Hämolyse | | | | | | | | | |

Alle in Tabelle 4 dargestellten Formulierungen sind sehr mild. Darüber hinaus wird deutlich, daß die Formulierungen am mildesten sind, die den größten Anteil C₈/C₁₀-Betain enthalten. Bei den Formulierungen F bis H geht dies aus den Denaturierungswerten (D) hervor, bei den Formulierungen I bis L sowohl aus den Lyse- (L) als auch aus den Denaturierungswerten.

### Beispiel 5

### Rezepturen

| Gesichtswasser | |
|---|---|
| Wasser | 85,5 % |
| Ethanol | 5,0 % |
| C₈/C₁₀-Betain ¹⁾ | 3,7 % |
| Feuchthaltemittel ²⁾ | 2,0 % |
| Hamamelisextrakt ³⁾ | 2,0 % |
| Glycerin-(6)-polyglykolether-Capryl-/Caprinsäureester ⁴⁾ | 1,5 % |
| Allantoin | 0,3 % |

| | |
|---|---|
| 1) 50 %ige wäßrige Lösung | |
| 2) CTFA-Bezeichnung: Sodium Lactate (and) Sodium PCA (and) Glycine (and) Fructose (and) Urea (and) Niacinamid (and) Inositol (and) Sodium Benzoate (and) Lactic Acid, im Handel erhältlich unter der Bezeichnung LACTIL (Th. Goldschmidt AG) | |
| 3) im Handel erhältlich unter der Bezeichnung Extrapon Hamamelis (Dragoco) | |
| 4) CTFA-Bezeichnung: PEG-6 Caprylic-/Capric Glycerides, im Handel erhältlich unter der Bezeichnung TEGOSOFT GMC6 (Th. Goldschmidt AG) | |

| Konditioniershampoo | |
|---|---|
| Wasser | 43,1 % |
| Natriumlaurylethersulfat (28 %ige wäßrige Lösung) ⁵⁾ | 32,0 % |
| C₈/C₁₀-Betain ¹⁾ | 22,0 % |
| Verdickungsmittel auf Polyacrylatbasis ⁶⁾ | 2,0 % |
| kationisches Guar ⁷⁾ | 0,5 % |
| kationisches Siloxan (50 %ige Lösung in Propylenglykol) ⁸⁾ | 0,4 % |
| Parfüm | 0,3 % |

| | |
|---|---|
| 1) 50 %ige wäßrige Lösung | |
| 5) CTFA-Bezeichnung: Sodium Laureth Sulfate, im Handel erhältlich unter der Bezeichnung Texapon N 25 (Henkel) | |
| 6) CTFA-Bezeichnung: Acrylates Steareth-50 Acrylate Copolymer (and) Laureth-3 (and) Propylene Glycol, im Handel erhältlich unter der Bezeichnung ANTIL 208 (Th. Goldschmidt AG) | |
| 7) CTFA-Bezeichnung: Guar Hydroxypropyl Trimonium Chloride, im Handel erhältlich unter der Bezeichnung Cosmedia Guar C261 (Henkel) | |
| 8) CTFA-Bezeichnung: Quaternium 80, im Handel erhältlich unter der Bezeichnung ABIL Quat 3272 (Th. Goldschmidt AG) | |

| Duschbad | |
|---|---|
| Wasser | 40,0 % |
| Dinatriumlaurylethersulfosuccinat ⁹⁾ | 17,3 % |
| C₈/C₁₀-Betain ¹⁾ | 17,0 % |
| Laurylpolyglucosid ¹⁰⁾ | 14,0 % |
| Glycerin-(7)-polyglykolether-Cocosfettsäureester ¹¹⁾ | 5,0 % |
| Verdickungsmittel auf Polyacrylatbasis ⁶⁾ | 4,0 % |
| NaCl | 2,5 % |
| Parfüm | 0,2 % |

| | |
|---|---|
| 1) 50 %ige wäßrige Lösung | |
| 6) CTFA-Bezeichnung: Acrylates Steareth-50 Acrylate Copolymer (and) Laureth-3 (and) Propylene Glycol, im Handel erhältlich unter der Bezeichnung ANTIL 208 (Th. Goldschmidt AG) | |
| 9) CTFA-Bezeichnung: Disodium Laurethsulfosuccinate, im Handel erhältlich unter der Bezeichnung TEGO SULFOSUCCINATE F 30 (Th. Goldschmidt AG) | |
| 10) CTFA-Bezeichnung: Lauryl Polyglucose, im Handel erhältlich unter der Bezeichnung Plantaren 1200 (Henkel) | |
| 11) CTFA-Bezeichnung: PEG-7 Glyceryl Cocoate, im Handel erhältlich unter der Bezeichnung TEGOSOFT GC (Th. Goldschmidt AG) | |

| Babyshampoo | |
|---|---|
| Wasser | 69,5 % |
| Natriumlaurylethersulfat (28 %ige wäßrige Lösung) ⁵⁾ | 15,0 % |
| Cocosfettsäureamidopropylbetain ¹²⁾ | 6,0 % |
| Glycerin-(18)-polyglykolether-Ölsäure/Cocosfettsäure ester ¹³⁾ | 4,0 % |
| Glycerin-(30)-polyglykolether-Talgfettsäureester ¹⁴⁾ | 3,0 % |
| C₈/C₁₀-Betain ¹⁾ | 1,5 % |
| NaCl | 0,7 % |
| Parfüm | 0,3 % |

| | |
|---|---|
| 1) 50 %ige wäßrige Lösung | |
| 5) CTFA-Bezeichnung: Sodium Laureth Sulfate, im Handel erhältlich unter de | |
| 12) CTFA-Bezeichnung: Cocamidopropyl Betaine, im Handel erhältlich unter der Bezeichnung TEGO Betain F 50 (Th. Goldschmidt AG) | |
| 13) CTFA-Bezeichnung: PEG-18 Glyceryl Oleate/Cocoate, im Handel erhältl Goldschmidt AG) | |
| 14) CTFA-Bezeichnung: PEG-30 Glyceryl Stearate, im Handel | |

## Patentansprüche

1. Milde, wäßrige, tensidische Zubereitungen für kosmetische Zwecke und Reinigungsmittel, dadurch gekennzeichnet, daß sie als Tenside Betaine der allgemeinen Formel 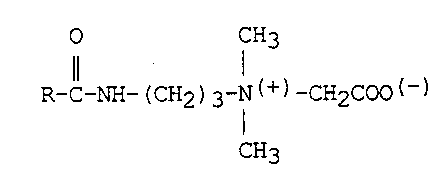 in der R einen Alkylrest mit 7 bis 21 C-Atomen bedeutet, wobei 20 bis 100 Gew.-% der eingesetzten Betaine als Rest R einen Alkylrest mit 7 bis 9 C-Atomen aufweisen, in einer Menge von 1,0 bis 40 Gew.-%, bezogen auf die Zubereitung, enthalten.

2. Zübereitungen nach Anspruch 1, dadurch gekennzeichnet, daß sie die Betaine in einer Menge von 1,5 bis 20 Gew.-%, bezogen auf die Zubereitung, enthalten.

3. Zubereitungen nach Anspruch 1 und 2, dadurch gekennzeichnet, daß 40 bis 100 Gew.-% der eingesetzten Betaine als Rest R einen Alkylrest mit 7 bis 9 C-Atomen aufweisen.

4. Zubereitungen nach Anspruch 1 bis 3, dadurch gekennzeichnet, daß mehr als 90 Gew.-% der eingesetzten Betaine als Rest R einen Alkylrest mit 7 bis 9 C-Atomen aufweisen.

5. Zubereitungen nach Anspruch 1 bis 4, dadurch gekennzeichnet, daß sie zusätzlich anionische und/oder nichtionische Tenside in einer Menge von 1,0 bis 40 Gew.-%, bezogen auf die Zubereitung, enthalten, mit der Maßgabe, daß der Gesamttensidgehalt nicht mehr als 60 Gew.-% beträgt.

## Claims

1. Mild, aqueous, surface-active preparations for cosmetic purposes and cleansers, characterized in that they comprise, as surfactants, betaines of the general formula 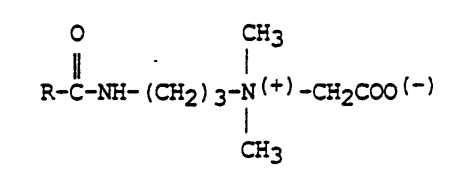 in which R is an alkyl radical having from 7 to 21 carbon atoms, where from 20 to 100% by weight of the betaines used have, as radical R, an alkyl radical having from 7 to 9 carbon atoms, in an amount of from 1.0 to 40% by weight, based on the preparation.

2. Preparations according to Claim 1, characterized in that they comprise the betaines in an amount of from 1.5 to 20% by weight, based on the preparation.

3. Preparations according to Claim 1 and 2, characterized in that from 40 to 100% by weight of the betaines used have, as radical R, an alkyl radical having from 7 to 9 carbon atoms.

4. Preparations according to Claims 1 to 3, characterized in that more than 90% by weight of the betaines used have, as radical R, an alkyl radical having from 7 to 9 carbon atoms.

5. Preparations according to Claims 1 to 4, characterized in that they additionally comprise anionic and/or nonionic surfactants in an amount of from 1.0 to 40% by weight, based on the preparation, with the proviso that the total surfactant content is not more than 60% by weight.

## Revendications

1. Préparations tensio-actives aqueuses douces pour usage cosmétique et agents de nettoyage, caractérisées en ce qu'elles contiennent, en tant que tensio-actifs, des bétaïnes de formule générale 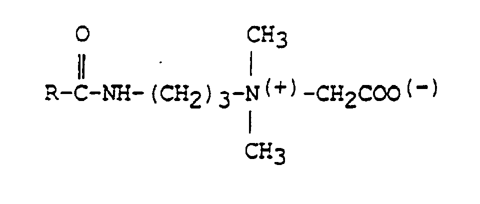 dans laquelle R représente un radical alkyle ayant de 7 à 21 atomes de carbone, où de 20 a 100% en poids des bétaïnes utilisées renferment en tant que radical R, un radical alkyle ayant de 7 à 9 atomes de carbone, en une quantité allant de 1,0 à 40% en poids, par rapport à la préparation.

2. Préparations selon la revendication 1, caractérisées en ce qu'elles contiennent des bétaïnes en une quantité allant de 1,5 à 20% en poids par rapport à la préparation.

3. Préparations selon les revendications 1 et 2, caractérisées en ce que de 40 à 100% en poids des bétaïnes utilisées renferment, en tant que radical R, un radical alkyle ayant de 7 à 9 atomes de carbone.

4. Préparations selon les revendications 1 à 3, caractérisées en ce que plus de 90% en poids des bétaïnes utilisées renferment, en tant que radical R, un radical alkyle ayant de 7 à 9 atomes de carbone.

5. Préparations selon les revendications 1 à 4, caractérisées en ce qu'elles contiennent en outre des tensio-actifs anioniques et/ou non ioniques en une quantité allant de 1,0 à 40% en poids, par rapport à la préparation, à condition que la teneur totale en tensio-actifs ne soit pas supérieure à 60% en poids.
